(19) **Europäisches Patentamt / European Patent Office / Office européen des brevets**

(11) **EP 4 494 748 A1**

(12) **EUROPEAN PATENT APPLICATION**
published in accordance with Art. 153(4) EPC

(43) Date of publication:
**22.01.2025 Bulletin 2025/04**

(21) Application number: 23770521.5

(22) Date of filing: **06.03.2023**

(51) International Patent Classification (IPC):
**B01D 61/36** (2006.01)        **B01D 71/70** (2006.01)

(52) Cooperative Patent Classification (CPC):
**B01D 61/36; B01D 71/70**

(86) International application number:
**PCT/JP2023/008428**

(87) International publication number:
**WO 2023/176565 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.03.2022 JP 2022043131**

(71) Applicant: **NITTO DENKO CORPORATION
Ibaraki-shi
Osaka 567-8680 (JP)**

(72) Inventors:
• **OGAWA, Tomoya**
  **Ibaraki-shi, Osaka 567-8680 (JP)**
• **KIMURA, Naomichi**
  **Ibaraki-shi, Osaka 567-8680 (JP)**
• **HIRAI, Tomoya**
  **Ibaraki-shi, Osaka 567-8680 (JP)**

(74) Representative: **Hoffmann Eitle
Patent- und Rechtsanwälte PartmbB
Arabellastraße 30
81925 München (DE)**

(54) **MEMBRANE SEPARATION SYSTEM AND METHOD FOR OPERATING MEMBRANE SEPARATION DEVICE**

(57)     The present invention provides a membrane separation system suitable for suppressing a decrease in a content of an organic compound in a permeated fluid. A membrane separation system 100 of the present invention includes a membrane separation device 10. The membrane separation device 10 has a separation membrane 11, and a feed space and a permeation space separated from each other by the separation membrane 11. When an aqueous solution S containing a volatile organic compound C is supplied to the feed space and the permeation space is decompressed, the separation membrane 11 separates the aqueous solution S into a permeated fluid 80 having a content of the organic compound C higher than that in the aqueous solution S and a non-permeated fluid 81 having a content of the organic compound C lower than that in the aqueous solution S. The membrane separation system 100 performs a pressurizing procedure that increases a pressure in the permeation space when at least one condition selected from the group consisting of conditions (A1) to (A3) is satisfied.

FIG.1

## Description

TECHNICAL FIELD

The present invention relates to a membrane separation system and a method for operating a membrane separation device.

BACKGROUND ART

There have been developed methods for producing a volatile organic compound (a fermented product), such as an alcohol, by fermenting a carbon source, such as glucose, by using a microorganism. The fermentation of a carbon source is carried out in an aqueous solution, for example. In this method, the fermentation by a microorganism stops in some cases when a content of the fermented product in the aqueous solution increases. In such cases, in order to continue the production of the fermented product by a microorganism, it is necessary to separate the fermented product from the aqueous solution while the fermentation is being carried out.

As an example of the method for separating a volatile organic compound from an aqueous solution containing the organic compound, a pervaporation method using a separation membrane can be mentioned. The pervaporation method is suitable for separating a volatile organic compound from an aqueous solution containing various substances. By taking advantage of this feature and combining a membrane separation device that performs the pervaporation method with a fermenter that produces a fermented product, it is possible to produce a fermented product continuously. For example, Patent Literature 1 discloses a membrane separation system obtained by combining a membrane separation device with a fermenter.

CITATION LIST

Patent Literature

Patent Literature 1: JP 2010-161987 A

SUMMARY OF INVENTION

Technical Problem

When a membrane separation device is operated continuously, a content of an organic compound in an aqueous solution to be introduced to the membrane separation device decreases in some cases. The decrease in the content occurs obviously in the case where the aqueous solution is a fermented liquid produced in a fermenter. A decrease in the content of the organic compound in the aqueous solution tends to decrease significantly a content of the organic compound in a permeated fluid.

In a membrane separation system, a separation tank that phase-separates a permeated fluid that is a liquid into a aqueous phase and an organic phase is used in some cases. However, when a content of an organic compound in the permeated fluid decreases, it is difficult to perform the phase separation of the permeated fluid in the separation tank. Furthermore, in the case where the membrane separation system includes a condensation unit for cooling and condensing the permeated fluid that is a gas, a decrease in the content of the organic compound in the permeated fluid not only lowers the energy efficiency of the condensation but also causes, in some cases, the permeated fluid to be frozen inside the condensation unit. As just described above, a decrease in the content of the organic compound in the permeated fluid can cause a problem in operation of the membrane separation system.

Therefore, the present invention is intended to provide a membrane separation system suitable for suppressing a decrease in a content of an organic compound in a permeated fluid.

Solution to Problem

The present invention provides a membrane separation system including a membrane separation device, wherein

the membrane separation device has a separation membrane, and a feed space and a permeation space separated from each other by the separation membrane,
when an aqueous solution containing a volatile organic compound is supplied to the feed space and the permeation space is decompressed, the separation membrane separates the aqueous solution into a permeated fluid having a content of the organic compound higher than that in the aqueous solution and a non-permeated fluid having a content of the organic compound lower than that in the aqueous solution, and
the membrane separation system performs a pressurizing procedure that increases a pressure in the permeation space when at least one condition selected from the group consisting of conditions (A1) to (A3) below is satisfied:

(A1) A content of the organic compound in the aqueous solution to be supplied to the feed space is below a first set value;
(A2) A content of the organic compound in the permeated fluid is below a second set value;
(A3) A content of the organic compound in the non-permeated fluid is below a third set value.

The present invention further provides a method for operating a membrane separation device having a separation membrane, and a feed space and a permeation space separated from each other by the separation membrane, wherein

when an aqueous solution containing a volatile organic compound is supplied to the feed space and the permeation space is decompressed, the separation membrane separates the aqueous solution into a permeated fluid having a content of the organic compound higher than that in the aqueous solution and a non-permeated fluid having a content of the organic compound lower than that in the aqueous solution, and
the operating method includes performing a pressurizing procedure that increases a pressure in the permeation space when at least one condition selected from the group consisting of conditions (A1) to (A3) below is satisfied:

(A1) A content of the organic compound in the aqueous solution to be supplied to the feed space is below a first set value;
(A2) A content of the organic compound in the permeated fluid is below a second set value;
(A3) A content of the organic compound in the non-permeated fluid is below a third set value.

Advantageous Effects of Invention

The present invention can provide a membrane separation system suitable for suppressing a decrease in a content of an organic compound in a permeated fluid.

BRIEF DESCRIPTION OF DRAWINGS

FIG. 1 is a schematic configuration diagram of a membrane separation system of the present embodiment.
FIG. 2 is a schematic cross-sectional view showing an example of a membrane separation device.
FIG. 3 is a schematic cross-sectional view of a separation membrane included in the membrane separation device.
FIG. 4 is a schematic cross-sectional view showing another example of the membrane separation device.
FIG. 5 is a graph showing the results of Measurement Examples 1 to 5.
FIG. 6 is a graph showing the results of Measurement Examples 6 to 8.

DESCRIPTION OF EMBODIMENTS

A membrane separation system according to a first aspect of the present invention is a membrane separation system including a membrane separation device, wherein

the membrane separation device has a separation membrane, and a feed space and a permeation space separated from each other by the separation membrane,
when an aqueous solution containing a volatile organic compound is supplied to the feed space and the permeation space is decompressed, the separation membrane separates the aqueous solution into a permeated fluid having a content of the organic compound higher than that in the aqueous solution and a non-permeated fluid having a content of the organic compound lower than that in the aqueous solution, and
the membrane separation system performs a pressurizing procedure that increases a pressure in the permeation space when at least one condition selected from the group consisting of conditions (A1) to (A3) below is satisfied:

(A1) A content of the organic compound in the aqueous solution to be supplied to the feed space is below a first set value;
(A2) A content of the organic compound in the permeated fluid is below a second set value;
(A3) A content of the organic compound in the non-permeated fluid is below a third set value.

According to a second aspect of the present invention, for example, the membrane separation system according to the first aspect performs, after performing the pressurizing procedure, a decompressing procedure that decreases the pressure in the permeation space when at least one condition selected from the group consisting of conditions (B1) to (B3) below is satisfied:

(B1) The content of the organic compound in the aqueous solution to be supplied to the feed space is above a fourth set value;
(B2) The content of the organic compound in the permeated fluid is above a fifth set value;
(B3) The content of the organic compound in the non-permeated fluid is above a sixth set value.

According to a third aspect of the present invention, for example, the membrane separation system according to the first or second aspect further includes a tank for storing the aqueous solution to be supplied to the membrane separation device.
According to a fourth aspect of the present invention, for example, the membrane separation system according to the third aspect further includes a non-permeated fluid discharge passage connected to the membrane separation device and configured to discharge the non-permeated fluid from the membrane separation device, wherein the non-permeated fluid discharge passage is connected to the tank.
According to a fifth aspect of the present invention, for example, in the membrane separation system according to the third or fourth aspect, the organic compound is a fermented product generated by a microorganism, and the tank is a fermenter for generating the organic compound.

According to a sixth aspect of the present invention, for example, the membrane separation system according to any one of the first to fifth aspects further includes a separation tank for phase-separating the permeated fluid that is a liquid into an aqueous phase containing water as a main component and an organic phase having a content of the organic compound higher than that in the aqueous phase.

According to a seventh aspect of the present invention, for example, in the membrane separation system according to any one of the first to sixth aspects, the separation membrane has a separation functional layer including a hydrophobic material.

According to an eighth aspect of the present invention, for example, in the membrane separation system according to the seventh aspect, the hydrophobic material includes a compound having a siloxane bond.

According to a ninth aspect of the present invention, for example, in the membrane separation system according to the seventh or eighth aspect, the separation functional layer includes a matrix including the hydrophobic material and a filler dispersed in the matrix.

According to a tenth aspect of the present invention, for example, in the membrane separation system according to the ninth aspect, the filler includes zeolite.

According to an eleventh aspect of the present invention, for example, in the membrane separation system according to any one of the first to tenth aspects, the organic compound is at least one selected from the group consisting of an alcohol, ketone, and ester.

According to a twelfth aspect of the present invention, for example, in the membrane separation system according to any one of the first to eleventh aspects, the organic compound is n-butanol.

A method for operating a membrane separation device according to a thirteenth aspect of the present invention is a method for operating a membrane separation device having a separation membrane, and a feed space and a permeation space separated from each other by the separation membrane, wherein

when an aqueous solution containing a volatile organic compound is supplied to the feed space and the permeation space is decompressed, the separation membrane separates the aqueous solution into a permeated fluid having a content of the organic compound higher than that in the aqueous solution and a non-permeated fluid having a content of the organic compound lower than that in the aqueous solution, and

the operating method includes performing a pressurizing procedure that increases a pressure in the permeation space when at least one condition selected from the group consisting of conditions (A1) to (A3) below is satisfied:

(A1) A content of the organic compound in the aqueous solution to be supplied to the feed space is below a first set value;
(A2) A content of the organic compound in the permeated fluid is below a second set value;
(A3) A content of the organic compound in the non-permeated fluid is below a third set value.

The present invention will be described in detail below. The following description is not intended to limit the present invention to a specific embodiment.

[Membrane separation system]

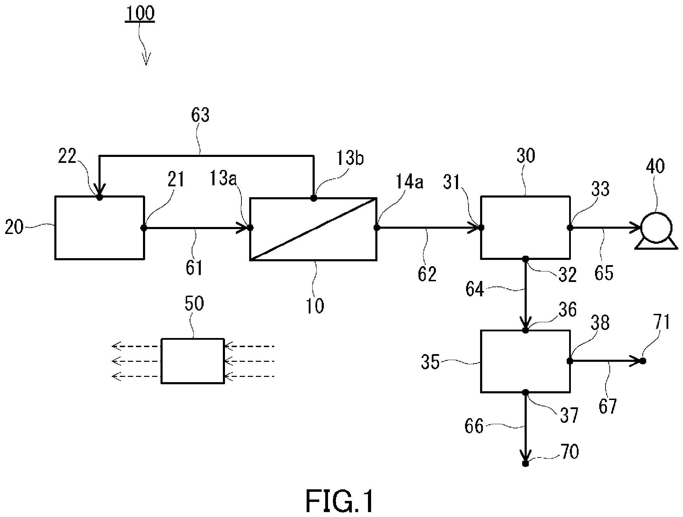

As shown in FIG. 1, a membrane separation system 100 of the present embodiment includes a membrane separation device 10. The membrane separation device 10 has a separation membrane, and a feed space and a permeation space separated from each other by the separation membrane. The membrane separation device 10 is a device that performs membrane separation, during its operation, for an aqueous solution S containing a volatile organic compound C by using the separation membrane.

The separation membrane that the membrane separation device 10 has can separate the aqueous solution S into a permeated fluid and a non-permeated fluid when the aqueous solution S is supplied to the feed space and the permeation space is decompressed. The separation membrane is a membrane that allows the organic compound C contained in the aqueous solution S to preferentially permeate therethrough. Accordingly, the permeated fluid separated by the separation membrane has a content of the organic compound C higher than that in the aqueous solution S. In contrast, the non-permeated fluid has a content of the organic compound C lower than that in the aqueous solution S. Note that, as described later, the organic compound C may be a fermented product generated by fermentation of a carbon source by a microorganism, or may be an alcohol (a bioalcohol, especially biobutanol), such as n-butanol, generated by a microorganism. That is, the aqueous solution S may be a fermented liquid containing the organic compound C as the fermented product.

The membrane separation system 100 further includes a tank 20 in addition to the membrane separation device 10. The tank 20 stores the aqueous solution S to be supplied to the membrane separation device 10. The tank 20 is typically a fermenter for generating the organic compound C by fermentation of a carbon source by a microorganism.

The membrane separation system 100 may further include a condensation unit 30 for condensing the permeated fluid that is a gas, and a decompression device 40. The condensation unit 30 is provided with, for example, a heat exchanger for cooling the permeated fluid. The heat exchanger is, for example, a gas-liquid heat exchanger that causes heat exchange between a cooling medium, such as an antifreeze, and the permeated fluid. In the condensation unit 30, the permeated fluid is cooled by the heat exchanger to form the permeated fluid that is a liquid. The condensation unit 30 may include a tank for storing the permeated fluid that is a liquid.

The decompression device 40 can decompress an inside of the permeation space of the membrane separation device 10 via the condensation unit 30, for example. Preferably, the decompression device 40 is a vacuum device such as a vacuum pump. The vacuum pump is typically a gas transport vacuum pump, and a reciprocating vacuum pump and a rotary vacuum pump, etc. can be mentioned. Examples of the reciprocating vacuum pump include a diaphragm vacuum pump and a rocking piston vacuum pump. Examples of the rotary vacuum pump include: a liquid seal pump; an oil rotary pump (a rotary pump); a mechanical booster pump; and various kinds of dry pumps such as a roots dry pump, a claw dry pump, a screw dry pump, a turbo dry pump, and a scroll dry pump. The pump as the decompression device 40 may include a variable speed mechanism for changing a rotational speed, etc. An example of the variable speed mechanism is an inverter that drives a motor of the pump. By controlling the rotational speed, etc. of the pump by the variable speed mechanism, it is possible to adjust properly a pressure in the permeation space of the membrane separation device 10.

The membrane separation system 100 may further include a separation tank 35 for phase-separating the permeated fluid condensed in the condensation unit 30. For example, when the permeated fluid that is a liquid is left at rest in the separation tank 35, the permeated fluid is phase-separated into an aqueous phase containing water as a main component and an organic phase having a content of the organic compound C higher than that in the aqueous phase. Note that the "main component" means a component having a largest content in the aqueous phase in terms of weight ratio. The aqueous phase may be, for example, a state in which the organic compound C is contained in addition to water and the organic compound C is saturated. The organic phase may include water even more in addition to the organic compound C.

The membrane separation system 100 further includes an aqueous solution feed passage 61, a permeated fluid discharge passage 62, and a non-permeated fluid discharge passage 63. The aqueous solution feed passage 61 is a passage connected to an aqueous solution outlet (an outlet 21) of the tank 20 and an aqueous solution inlet (an inlet 13a) of the membrane separation device 10 and configured to supply the aqueous solution S from the tank 20 to the membrane separation device 10. The aqueous solution feed passage 61 may be provided with a pump that controls a flow rate of the aqueous solution S, and may be provided with a sensor for measuring the content of the organic compound C in the aqueous solution S.

The permeated fluid discharge passage 62 is a passage connected to a permeated fluid outlet (an outlet 14a) of the membrane separation device 10 and a permeated fluid inlet (an inlet 31) of the condensation unit 30 and configured to deliver the permeated fluid from the membrane separation device 10 to the condensation unit 30. The permeated fluid discharge passage 62 may be provided with a sensor for measuring the content of the organic compound C in the permeated fluid.

The non-permeated fluid discharge passage 63 is a passage connected to a non-permeated fluid outlet (an outlet 13b) of the membrane separation device 10 and configured to discharge the non-permeated fluid from the membrane separation device 10. The non-permeated fluid discharge passage 63 may be provided with a sensor for measuring the content of the organic compound C in the non-permeated fluid.

The non-permeated fluid discharge passage 63 may be connected to a non-permeated fluid inlet (an inlet 22) of the tank 20 and configured to deliver the non-permeated fluid to the tank 20. That is, the membrane separation system 100 may be configured to allow the non-permeated fluid to be mixed with the aqueous solution S in the tank 20 and circulate through the aqueous solution feed passage 61 and the non-permeated fluid discharge passage 63. In the case where the non-permeated fluid is delivered to the tank 20, the aqueous solution S is mixed with the non-permeated fluid and the content of the organic compound C in the aqueous solution S decreases in the tank 20. In the case where the tank 20 is a fermenter, a decrease in the content of the organic compound C in the aqueous solution S can inhibit fermentation by a microorganism from stopping, thereby making it possible to produce a fermented product continuously.

The membrane separation system 100 further includes a liquid discharge passage 64 and a decompression passage 65. The liquid discharge passage 64 is a passage connected to a liquid outlet 32 of the condensation unit 30 and a liquid inlet 36 of the separation tank 35 and configured to deliver, from the condensation unit 30 to the separation tank 35, a permeated fluid 80 that is a liquid.

The decompression passage 65 is connected to a gas outlet 33 of the condensation unit 30 and the decompression device 40. The decompression device 40 can decompress the inside of the permeation space of the membrane separation device 10 via the decompression passage 65, the condensation unit 30, and the permeated fluid discharge passage 62.

The membrane separation system 100 further includes an aqueous phase discharge passage 66 and an organic phase discharge passage 67. The aqueous phase discharge passage 66 is a passage connected to an aqueous phase outlet 37 of the separation tank 35 and configured to discharge, from the separation tank 35, the aqueous phase generated in the separation tank 35. In the aqueous phase discharge passage 66, an opening (a discharge outlet 70) for discharging the aqueous phase from the aqueous phase discharge passage 66 is formed. The aqueous phase discharge passage 66 may be connected to a tank that stores the aqueous phase, or a distillation unit (a distillation column, for example) that performs a distillation treatment for the aqueous phase.

The organic phase discharge passage 67 is a passage connected to an organic phase outlet 38 of the separation tank 35 and configured to discharge, from the separation tank 35, the organic phase generated in the separation tank 35. In the organic phase discharge passage 67, an opening (a discharge outlet 71) for discharging the organic phase from the organic phase discharge passage 67 is formed. The organic phase discharge passage 67 may be connected to a tank that stores the organic phase, or a distillation unit (a distillation column, for example) that performs a distillation treatment for the organic phase.

The membrane separation system 100 may further include a controller 50 that controls each member of the membrane separation system 100. The controller 50 is, for example, a DSP (Digital Signal Processor) including an A/D conversion circuit, an input/output circuit, an arithmetic circuit, a storage device, etc. A program for operating properly the membrane separation system 100 is stored in the controller 50. For example, the controller 50 can receive information about the content of the organic compound C from each of the sensors that the passages, such as the aqueous solution feed passage 61, the permeated fluid discharge passage 62, and the non-permeated fluid discharge passage 63, are provided with and can control behavior of the decompression device 40 based on the information. Specifically, the controller 50 can control behavior of the decompression device 40 so as to perform the later-described pressurizing procedure and decompressing procedure during operations of the membrane separation system 100 and the membrane separation device 10.

Each passage of the membrane separation system 100 is composed of, for example, a metal or resin pipe unless otherwise noted.

[Membrane separation device]

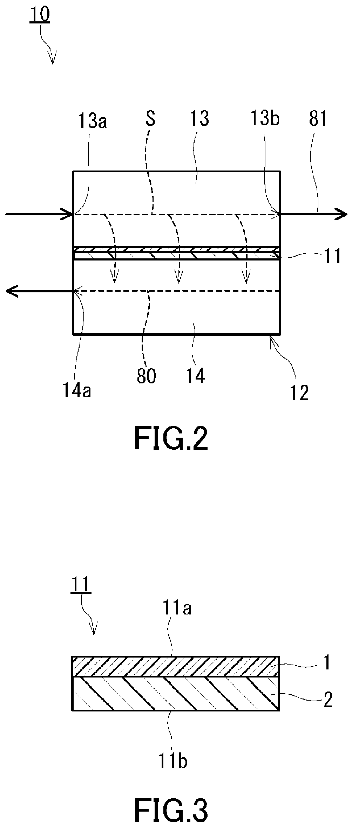

As shown in FIG. 2, the membrane separation device 10 includes a separation membrane 11 and a tank 12. The tank 12 has a first chamber 13 and a second chamber 14. The first chamber 13 functions as the feed space to which the aqueous solution S is supplied. The second chamber 14 functions as the permeation space to which the permeated fluid 80 is supplied. The permeated fluid 80 is obtained by allowing the aqueous solution S to permeate through the separation membrane 11. The separation membrane 11 is disposed in the tank 12. In the tank 12, the separation membrane 11 separates the first chamber 13 from the second chamber 14. The tank 12 has a pair of wall surfaces, and the separation membrane 11 extends from one of them to the other.

The first chamber 13 has the inlet 13a and the outlet 13b. The second chamber 14 has the outlet 14a. The inlet 13a of the first chamber 13 is an opening for supplying the aqueous solution S to the membrane separation device 10. The outlet 14a of the second chamber 14 is an opening for discharging the permeated fluid 80 from the membrane separation device 10. The outlet 13b of the first chamber 13 is an opening for discharging, from the membrane separation device 10, the aqueous solution S (a non-permeated fluid 81) not having permeated through the separation membrane 11. The inlet 13a, the outlet 13b, and the outlet 14a are formed, for example, in wall surfaces of the tank 12.

The membrane separation device 10 is suitable for a flow-type (continuous-type) membrane separation method. However, the membrane separation device 10 may be used for a batch-type membrane separation method.

(Separation membrane)

The separation membrane 11 is not particularly limited as long as the separation membrane 11 allows the organic compound C contained in the aqueous solution S to preferentially permeate therethrough. The separation membrane 11 is, for example, a pervaporation membrane that allows the permeated fluid 80 that is a gas and contains the organic compound C to be generated by a pervaporation method.

As shown in FIG. 3, the separation membrane 11 includes, for example, a separation functional layer 1 and a porous support member 2 supporting the separation functional layer 1. The separation membrane 11 may further include a protective layer (not shown) that protects the separation functional layer 1. The separation functional layer 1 is in direct contact with the porous support member 2, for example. For example, the separation membrane 11 has a principal surface 11a, on the separation functional layer side, that is exposed to the first chamber 13 and a principal surface 11b, on the porous support member side, that is exposed to the second chamber 14.

(Separation functional layer)

The separation functional layer 1 is a layer that allows the organic compound C contained in the aqueous solution S to preferentially permeate therethrough. The separation functional layer 1 includes a hydrophobic material, for example. In the present description, the term "hydrophobic material" refers to, for example, a material that has a static contact angle exceeding 90° with respect to water when a 10 μL drop of the water (temperature 25°C) is dropped on a surface of a specimen composed of the material. Note that the static contact angle with respect to water can be measured using a commercially available contact angle meter.

Examples of the hydrophobic material include a compound having a siloxane bond (a Si-O-Si bond), an olefin-based polymer, an oil, and a fluorine-based compound. The separation functional layer 1 preferably includes a compound having a siloxane bond as the hydrophobic material. The compound having a siloxane bond is typically a silicone-based polymer. The silicone-based polymer may be a solid or a liquid at 25°C. Specific examples of the silicone-based polymer include polydimethylsiloxane (PDMS). Specific examples of the olefin-based polymer include polypropylene. Examples of the oil include a hydrocarbon-based oil such as liquid paraffin. Examples of the fluorine-based compound include polytetrafluoroethylene (PTFE), polyvinylidene fluoride (PVDF), and tetrafluoroethylene-perfluoroalkyl vinyl ether copolymer (PFA). These hydrophobic materials can be used alone or two or more of them can be used in combination.

The separation functional layer 1 may include the hydrophobic material as a main component, or may be composed substantially of the hydrophobic material alone. The "main component" means a component having a largest content in the separation functional layer 1 in terms of weight ratio.

The separation functional layer 1 may include a matrix including the hydrophobic material and a filler dispersed in the matrix. The filler is buried in the matrix. In the matrix, all particles of the filler may be spaced from each other or may aggregate partially.

The filler includes, for example, an inorganic material such as zeolite, silica, or bentonite. The zeolite included in the filler is preferably a high-silica zeolite having a high ratio of silica with respect to alumina. Having high resistance to hydrolysis, the high-silica zeolite is suitably used for separating the aqueous solution S. As the high-silica zeolite, HSZ (registered trademark) available from Tosoh Corporation, HiSiv (registered trademark) available from UNION SHOWA K.K., USKY available from UNION SHOWA K.K., and Zeoal (registered trademark) available from Nakamura Choukou Co., Ltd. can be used.

The filler may include a metal organic framework (MOF). The metal organic framework is also referred to as a porous coordination polymer (PCP). The metal organic framework is preferably hydrophobic. The metal organic framework includes a metal ion and an organic ligand, for example. Examples of the metal ion include a Zn ion. The organic ligand includes an aromatic ring, for example. Examples of the aromatic ring included in the organic ligand include an imidazole ring. Examples of the organic ligand include 2-methylimidazole. Specific examples of the metal organic framework include ZIF-8.

The filler is in the form of particles, for example. The term "form of particles" herein includes spherical, elliptical, flaky, and fibrous forms. An average particle diameter of the filler is, for example, but not particularly limited to, 50 μm or less, preferably 20 μm or less, and more preferably 10 μm or less. The lower limit of the average particle diameter of the filler is 0.01 μm, for example. The average particle diameter of the filler can be determined by the following method, for example. First, a cross-section of the separation functional layer 1 is observed using a transmission electron microscope. The area of a specific particle of the filler on the resulting electron microscope image is calculated by image processing. The diameter of a circle having the same area as the calculated area is regarded as the particle diameter (the diameter of the particle) of the specific filler particle. The particle diameter was calculated for any number (at least 50) of the filler particles, and the average of the calculated values was regarded as the average particle diameter of the filler.

A content of the filler in the separation functional layer 1 is, for example, 10 wt% or more, preferably 30 wt% or more, and more preferably 40 wt% or more. The upper limit of the content of the filler in the separation functional layer 1 is, for example, but not particularly limited to, 70 wt%. A content of the matrix in the separation functional layer 1 is, for example, but not particularly limited to, 30 wt% to 90 wt%.

The separation functional layer 1 has a thickness of, for example, 200 μm or less, preferably 100 μm or less, and more preferably 80 μm or less. The separation functional layer 1 may have a thickness of 1.0 μm or more, 10 μm or more, and 30 μm or more.

The separation functional layer 1 may have a microporous structure with an average pore diameter of less than 0.01 μm, but may be a dense layer having no pore on its surface.

(Porous support member)

Examples of the porous support member 2 include: a nonwoven fabric; porous polytetrafluoroethylene; aromatic polyamide fiber; a porous metal; a sintered metal; porous ceramic; porous polyester; porous nylon; activated carbon fiber; latex; silicone; silicone rubber; a permeable (porous) polymer including at least one selected from the group consisting of polyvinyl fluoride, polyvinylidene fluoride, polyurethane, polypropylene, polyethylene, polystyrene, polycarbonate, polysulfone, polyether ether ketone, polyacrylonitrile, polyimide, and polyphenylene oxide; a metallic foam having an open cell or a closed cell; a polymer foam having an open cell or a closed cell; silica; a porous glass; and a mesh screen. The porous support member 2 may be a combination of two or more of these materials.

The porous support member 2 has an average pore diameter of 0.01 to 0.4 μm, for example. A thickness of the porous support 2 is, for example, but not particularly limited to, 10 μm or more, preferably 50 μm or more, and more preferably 100 μm or more. The thickness of the porous support 2 is, for example, 300 μm or less, and

preferably 200 $\mu$m or less.

(Protective layer)

The protective layer covers a surface of the separation functional layer 1, for example. A material of the protective layer is, for example, but not particularly limited to, a silicone resin. The material of the protective layer may be the same as that of the matrix of the separation functional layer 1. A thickness of the protective layer is, for example, but not particularly limited to, 5 $\mu$m or more, preferably 10 $\mu$m or more, and more preferably 20 $\mu$m or more. The thickness of the protective layer is, for example, 100 $\mu$m or less, and preferably 50 $\mu$m or less.

(Method for manufacturing separation membrane)

The separation membrane 11 can be produced by, for example, forming the separation functional layer 1 on the porous support member 2. Specifically, a coating liquid containing the materials of the separation functional layer 1 is prepared first. The coating liquid may contain, in addition to the filler, a dispersant for dispersing the filler in the coating liquid. In the case where the coating liquid contains the compound having a siloxane bond, the coating liquid may further contain a catalyst for curing the compound. Next, the coating liquid is applied onto the porous support 2 to obtain a coating. The coating is dried to obtain the separation functional layer 1.

(Characteristics of separation membrane)

A separation factor of the separation membrane 11 for the organic compound C with respect to water is not particularly limited. In one example, a separation factor $\alpha$ of the separation membrane 11 for n-butanol (BuOH) with respect to water may be 10 to 100. The separation factor $\alpha$ can be measured by the following method. First, in a state in which a liquid mixture composed of BuOH and water is in contact with one surface (the principal surface 11a, on the separation functional layer side, of the separation membrane 11, for example) of the separation membrane 11, a space adjacent to another surface (the principal surface 11b, on the porous support member side, of the separation membrane 11, for example) of the separation membrane 11 is decompressed. Thereby, a permeated fluid having permeated through the separation membrane 11 can be obtained. A weight ratio of the water and a weight ratio of the BuOH in the permeated fluid are measured. In the above procedure, a content of the BuOH in the liquid mixture is 0.5 wt%. The liquid mixture in contact with the separation membrane 11 has a temperature of 30°C. The space adjacent to the other surface of the separation membrane 11 is decompressed to 1.5 kPa. The separation factor $\alpha$ can be calculated by the following formula. Noted that in the following formula, $X_a$ and $X_b$ are respectively a weight ratio of the BuOH and a weight ratio of the water in the liquid mixture. $Y_a$ and $Y_b$ are respectively the weight ratio of the BuOH and the weight ratio of the water in the permeated fluid having permeated through the separation membrane 11.

$$\text{Separation factor } \alpha = (Y_a/Y_b)/(X_a/X_b)$$

Under the above measurement conditions for the separation factor $\alpha$, a flux of the BuOH permeating through the separation membrane 11 is, for example, but not particularly limited to, 0.01 (g/min/m$^2$) to 1.0 (g/min/m$^2$).

Under the above measurement conditions for the separation factor $\alpha$, a content of the BuOH in the permeated fluid having permeated through the separation membrane 11 is, for example, but not particularly limited to, 1 wt% to 20 wt%.

[Method for operating membrane separation system and membrane separation device]

(Normal operation)

A method for operating the membrane separation system 100 and the membrane separation device 10 is carried out as follows, for example. First, the aqueous solution S is supplied to the first chamber 13 of the membrane separation device 10 via the inlet 13a. This makes it possible to bring the aqueous solution S into contact with one surface (the principal surface 11a, for example) of the separation membrane 11.

The organic compound C included in the aqueous solution S is not particularly limited as long as it has volatility. In the present description, the term "organic compound having volatility" refers to an organic compound that has a boiling point of 20°C to 260°C, preferably 50 to 260°C, under an atmospheric pressure (101.325 kPa). Note that the organic compound C allows, for example, an aqueous phase containing water as a main component and an organic phase having a content of the organic compound C higher than that in the aqueous phase to be generated when a concentration of the organic compound C is high in the aqueous solution.

The number of carbon atoms in the organic compound C is, for example, but not particularly limited to, 10 or less, and may be 8 or less, 6 or less, or even 4 or less. The lower limit of the number of carbon atoms in the organic compound C may be 1 or 2. The organic compound C has, for example, a functional group including an oxygen atom, such as a hydroxyl group, a carbonyl group, an ether group, and an ester group. In the organic compound C, the number of the functional groups including an oxygen atom is typically one.

The organic compound C is, for example, at least one selected from the group consisting of an alcohol, ketone, and ester, and preferred is an alcohol. The alcohol may be an alkyl alcohol composed only of an alkyl group and a hydroxyl group, or may be an aryl alcohol including an aryl group and a hydroxyl group. The alkyl alcohol may be linear, branched, or cyclic. Examples of the alkyl alcohol include methanol, ethanol, n-propanol, isopropanol, n-butanol (BuOH), 2-butanol, isobutanol, t-butanol, and n-pentanol, and preferred is n-butanol. Examples of the aryl alcohol include phenol.

The ketone may be dialkylketone composed only of an alkyl group and a carbonyl group. Examples of the dialkylketone include methyl ethyl ketone (MEK) and acetone.

The ester may be aliphatic alkylester composed only of an alkyl group and an ester group. Examples of the aliphatic alkylester include ethyl acetate.

Note that the organic compound C is not limited to those mentioned above. The organic compound C may be aromatic hydrocarbon such as benzene, toluene, and xylene.

The aqueous solution S may contain one kind of the organic compound C, or two or more kinds of the organic compounds C. The content of the organic compound C in the aqueous solution S is, for example, 50 wt% or less, and may be 30 wt% or less, 10 wt% or less, 5 wt% or less, 2 wt% or less, or even 1 wt% or less. The lower limit of the content of the organic compound C is, for example, but not particularly limited to, 0.01 wt%.

The organic compound C may be a fermented product generated by fermentation of a carbon source by a microorganism, or may be an alcohol (a bioalcohol, especially biobutanol), such as n-butanol, generated by a microorganism. That is, the aqueous solution S may be a fermented liquid containing the organic compound C as the fermented product. However, the aqueous solution S is not limited to the fermented liquid and may be wastewater discharged from a chemical plant, etc.

The aqueous solution S may further contain an additional component, such as a microorganism, a carbon source, a nitrogen source, and an inorganic ion, other than water and the organic compound C. Examples of the microorganism include a bacteria such as Clostridium. Examples of the carbon source include polysaccharides such as starch, and monosaccharides such as glucose. A content of the additional component in the aqueous solution S is, for example, but not particularly limited to, 1 wt% to 10 wt%.

The amount of the aqueous solution S to be supplied to the membrane separation device 10 is not particularly limited and is determined according to the processing capacity of the membrane separation device 10. In one example, the amount of the aqueous solution S to be supplied is 0.5 kg/hr to 500 kg/hr. The aqueous solution S to be supplied to the first chamber 13 may be heated in advance. In one example, the aqueous solution S to be supplied to the first chamber 13 has a temperature of 30°C to 75°C.

Next, in a state in which the aqueous solution S is in contact with one surface of the separation membrane 11, a space adjacent to another surface (the principal surface 11b, for example) of the separation membrane 11 is decompressed. Specifically, an inside of the second chamber 14 is decompressed via the outlet 14a. The inside of the second chamber 14 can be decompressed by the decompression device 40, for example. A pressure in the second chamber 14 is, for example, 50 kPa or less, and may be 20 kPa or less, 10 kPa or less, 5 kPa or less, 3 kPa or less, or even 2 kPa or less. In the present description, the term "pressure" means an absolute pressure unless otherwise noted.

Decompressing the inside of the second chamber 14 makes it possible to obtain, on the side of the other surface of the separation membrane 11, the permeated fluid 80 having a high content of the organic compound C. In other words, the permeated fluid 80 is supplied to the second chamber 14. The permeated fluid 80 may be a gas or a liquid in the second chamber 14. The permeated fluid 80 is discharged outside the membrane separation device 10 via the outlet 14a. The permeated fluid 80 that is a gas is cooled in the condensation unit 30, etc., for example. Thereby, the permeated fluid 80 is liquefied to obtain the permeated fluid 80 that is a liquid.

On the other hand, the content of the organic compound C in the aqueous solution S gradually decreases from the inlet 13a toward the outlet 13b in the first chamber 13. The aqueous solution S (the non-permeated fluid 81) processed in the first chamber 13 is discharged outside the membrane separation device 10 via the outlet 13b. The non-permeated fluid 81 is typically a liquid.

As described above, the separation membrane 11 of the membrane separation device 10 allows the organic compound C contained in the aqueous solution S to preferentially permeate therethrough. Accordingly, the permeated fluid 80 obtained by the operation of the membrane separation device 10 has a content of the organic compound C higher than that in the aqueous solution S to be supplied to the membrane separation device 10. A ratio of the content (wt%) of the organic compound C in the permeated fluid 80 with respect to the content (wt%) of the organic compound C in the aqueous solution S is, for example, but not particularly limited to, 5 to 50.

(Pressurizing procedure)

As described above, the content of the organic compound C in the aqueous solution S to be introduced to the membrane separation device 10 decreases during the above-mentioned normal operation in some cases. When the content of the organic compound C in the aqueous solution S decreases, the content of the organic compound C in each of the permeated fluid 80 and the non-permeated fluid 81 also decreases. The membrane separation system 100 of the present embodiment performs a pressurizing procedure that increases a pressure in the permeation space when at least one condition selected from the group consisting of conditions (A1) to (A3) below is satisfied.

(A1) A content of the organic compound C in the aqueous solution S to be supplied to the feed space is below a first set value.

(A2) A content of the organic compound C in the permeated fluid 80 is below a second set value.

(A3) A content of the organic compound C in the non-permeated fluid 81 is below a third set value.

In other words, the method for operating the membrane separation device 10 includes performing the above pressurizing procedure when at least one condition selected from the group consisting of the above conditions (A1) to (A3) is satisfied.

The membrane separation system 100 preferably performs the pressurizing procedure when the condition (A1) out of the conditions (A1) to (A3) is satisfied. In the conditions (A1) to (A3), each of the set values can be set as appropriate in accordance with the type of the organic compound C, separation performance of the separation membrane 11, and operating conditions of the membrane separation system 100. The first set value is, for example, a value of 1.2 wt% or less, and may be a value of 1.1 wt% or less, 1.0 wt% or less, 0.9 wt% or less, 0.8 wt% or less, 0.7 wt% or less, 0.6 wt% or less, 0.5 wt% or less, or even 0.4 wt% or less. The first set value may be set within a range of 0.1 wt% to 1.2 wt%, 0.1 wt% to 0.7 wt%, or even 0.3 wt% to 0.5 wt%.

The second set value is, for example, a value of 3 wt% or more, and may be a value of 4 wt% or more, 5 wt% or more, 6 wt% or more, 7 wt% or more, 8 wt% or more, 9 wt% or more, 10 wt% or more, 11 wt% or more, 12 wt% or more, 13 wt% or more, or even 14 wt% or more. The second set value may be set within a range of 3 wt% to 30 wt%, 8 wt% to 30 wt%, or even 10 wt% to 25 wt%.

The third set value is, for example, a value of 1.2 wt% or less, and may be a value of 1.1 wt% or less, 1.0 wt% or less, 0.9 wt% or less, 0.8 wt% or less, 0.7 wt% or less, 0.6 wt% or less, 0.5 wt% or less, or even 0.4 wt% or less. The third set value may be set within a range of 0.08 wt% to 1.2 wt%, 0.08 wt% to 0.7 wt%, or even 0.1 wt% to 0.3 wt%.

Note that from the viewpoint of phase-separating easily, in the separation tank 35, the permeated fluid 80 that is a liquid, the content of the organic compound C (especially n-butanol) in the permeated fluid 80 is preferably 8 wt% or more, and especially preferably 10 wt% or more. Therefore, each of the set values of the conditions (A1) to (A3) is preferably set within a range that allows the content of the organic compound C in the permeated fluid 80 to be adjusted to 8 wt% or more, especially 10 wt% or more.

The pressurizing procedure is performed by increasing the pressure in the second chamber 14 (the permeation space) during the above-mentioned normal operation. In one example, when the pressure in the permeation space during the normal operation is less than 2 kPa (especially when the pressure is about 1.5 kPa), the membrane separation system 100 increases the pressure in the permeation space from a value of less than 2 kPa to a value of 2 kPa or more. In this case, the membrane separation system 100 may increase the pressure in the permeation space to a value of 3 kPa or more. In another example, when the pressure in the permeation space during the normal operation is less than 5 kPa, the membrane separation system 100 increases the pressure in the permeation space from a value of less than 5 kPa to a value of 5 kPa or more. In the pressurizing procedure, the membrane separation system 100 may increase the pressure in the permeation space to a value of 10 kPa or more, 20 kPa or more, or even 50 kPa or more. By the pressurizing procedure, the pressure in the permeation space may be adjusted to a value that allows the permeated fluid 80 to be generated in an amount that makes a composition of the permeated fluid 80 measurable, or may be adjusted to a value, such as an atmospheric pressure in a measurement environment, that allows the permeated fluid 80 to be hardly generated.

Each of the conditions (A1) to (A3) is a condition under which the content of the organic compound C in the permeated fluid 80 decreases. Therefore, by performing the pressurizing procedure when at least one of the conditions (A1) to (A3) is satisfied, it is possible to reduce a flow rate of the permeated fluid 80 having a low content of the organic compound C, and in some cases, it is possible to prevent the permeated fluid 80 from being generated. This makes it possible to prevent the permeated fluid 80 having a low content of the organic compound C from being delivered to the condensation unit 30 and the separation tank 35 and decreasing the content of the organic compound C in the permeated fluid 80 already present in the condensation unit 30 and the separation tank 35. As just described above, the membrane separation system 100 of the present embodiment is suitable for suppressing a decrease in the content of the organic compound C in the permeated fluid 80. Note that by suppressing a decrease in the content of the organic compound C in the permeated fluid 80 present in the separation tank 35, it is possible to phase-separate easily, in the separation tank 35, the permeated fluid 80 that is a liquid. By suppressing a decrease in the content of the organic compound C in the permeated fluid 80 delivered to the condensation unit 30, it is also possible to inhibit the permeated fluid from being frozen inside the condensation unit when the permeated fluid 80 that is a gas is cooled and condensed.

(Decompressing procedure)

Performing the above-mentioned pressurizing procedure tends to increase gradually the content of the organic compound C in the aqueous solution S to be introduced to the membrane separation device 10. This tendency is especially obvious in the case of a configuration in which the tank 20 is a fermenter and the non-permeated fluid 81 circulates through the aqueous solution feed passage 61 and the non-permeated fluid discharge passage 63. When the content of the organic compound C in the aqueous solution S increases, the content of the organic compound C in each of the permeated fluid 80 and the non-permeated fluid 81 tends to increase as well.

For example, the membrane separation system 100 of the present embodiment performs, after performing the above-mentioned pressurizing procedure, a decompressing procedure that decreases the pressure in the permeation space when at least one condition selected from the group consisting of conditions (B1) to (B3) below is satisfied.

(B1) The content of the organic compound C in the aqueous solution S to be supplied to the feed space is above a fourth set value.
(B2) The content of the organic compound C in the permeated fluid 80 is above a fifth set value.
(B3) The content of the organic compound C in the non-permeated fluid 81 is above a sixth set value.

In other words, the method for operating the membrane separation device 10 includes, for example, performing, after performing the pressurizing procedure, the above-mentioned decompressing procedure when at least one condition selected from the group consisting of the conditions (B1) to (B3) is satisfied.

The membrane separation system 100 preferably performs the decompressing procedure when the condition (B1) out of the conditions (B1) to (B3) is satisfied. In the conditions (B1) to (B3), each of the set values can be set as appropriate in accordance with the type of the organic compound C, separation performance of the separation membrane 11, and operating conditions of the membrane separation system 100. The fourth set value is, for example, a value of 0.08 wt% or more, and may be a value of 0.1 wt% or more, 0.2 wt% or more, or even 0.3 wt% or more. The fourth set value may be set within a range of 0.08 wt% to 1.2 wt%, 0.08 wt% to 0.7 wt%, or even 0.3 wt% to 0.5 wt%. The fourth set value may be equal to or different from the first set value.

The fifth set value is, for example, a value of 3 wt% or more, and may be a value of 4 wt% or more, 5 wt% or more, 6 wt% or more, 7 wt% or more, 8 wt% or more, 9 wt% or more, 10 wt% or more, 11 wt% or more, 12 wt% or more, 13 wt% or more, or even 14 wt% or more. The fifth set value may be set within a range of 3 wt% to 30 wt%, 8 wt% to 30 wt%, or even 10 wt% to 25 wt%.

The sixth set value is, for example, a value of 0.08 wt% or more, and may be a value of 0.1 wt% or more, 0.2 wt% or more, or even 0.3 wt% or more. The sixth set value may be set within a range of 0.08 wt% to 1.2 wt%, 0.08 wt% to 0.7 wt%, or even 0.3 wt% to 0.5 wt%.

The decompressing procedure is performed by decreasing the pressure in the second chamber 14 (the permeation space) after the pressurizing procedure. In the decompressing procedure, the membrane separation system 100 decreases the pressure in the permeation space to the pressure during the normal operation, for example. In one example, in the case where the pressure in the permeation space after the pressurizing procedure is 2 kPa or more, the membrane separation system 100 decreases the pressure in the permeation space from a value of 2 kPa or more to a value of less than 2 kPa (especially to a value of about 1.5 kPa). In another example, in the case where the pressure in the permeation space after the pressurizing procedure is 5 kPa or more, the membrane separation system 100 decreases the pressure in the permeation space from a value of 5 kPa or more to a value of less than 5 kPa.

Each of the conditions (B1) to (B3) is a condition under which the content of the organic compound C in the permeated fluid 80 increases. By performing the decompressing procedure when at least one of the conditions (B1) to (B3) is satisfied, it is possible to obtain the permeated fluid 80 having a high content of the organic compound C at a high flow rate. By combining the pressurizing procedure and the decompressing procedure, the membrane separation system 100 of the present embodiment makes it possible to suppress fluctuation in the content of the organic compound C in the permeated fluid 80.

<Modification of membrane separation device>

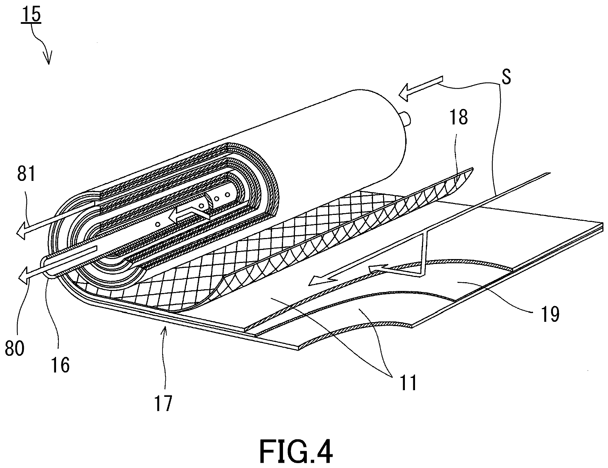

The membrane separation device 10 may be a spiral membrane element, a hollow fiber membrane element, a disk tube membrane element in which a plurality of pervaporation membranes are laminated, a plate-and-flame membrane element, or the like. FIG. 4 shows a spiral membrane element. A membrane separation device 15 of FIG. 4 includes a central tube 16 and a laminate 17. The laminate 17 includes the separation membrane 11.

The central tube 16 has a cylindrical shape. The central tube 16 has a surface with a plurality of holes or slits to allow the permeated fluid 80 to flow into the central tube 16. Examples of a material of the central tube 16 include: a resin such as an acrylonitrile-butadiene-styrene copolymer resin (an ABS resin), a polyphenylene ether resin (a PPE resin), or a polysulfone resin (a PSF resin); and a metal such as stainless steel or titanium. The central tube 16 has an inner diameter in a range of, for example, 20 to 100 mm.

The laminate 17 further includes a feed-side flow passage material 18 and a permeation-side flow passage material 19 in addition to the separation membrane 11. The laminate 17 is wound around the central tube 16. The membrane separation device 15 may be further provided with an exterior material (not shown).

As the feed-side flow passage material 18 and the permeation-side flow passage material 19, a resin net, woven fabric, or knitted fabric composed of polyethylene, polypropylene, polyethylene terephthalate (PET), polyphenylene sulfide (PPS), or an ethylene-chlorotrifluoroethylene copolymer (ECTFE) can be used, for example.

A method for operating the membrane separation device 15 is carried out as follows, for example. First, the aqueous solution S is supplied into an end of the wound laminate 17. A space inside the central tube 16 is decompressed. Thereby, the permeated fluid 80 having permeated through the separation membrane 11 of the laminate 17 moves into the central tube 16. The permeated fluid 80 is discharged outside via the central tube 16. The aqueous solution S processed by the membrane separation device 15 (the non-permeated fluid 81) is discharged outside from another end of the wound laminate 17.

EXAMPLES

Hereinafter, the present invention will be described in more detail by way of Examples and Comparative Examples, but the present invention is not limited to these examples.

[Production of separation membrane]

First, a separation membrane (a pervaporation membrane) was produced by the following method. A coating liquid was prepared by mixing 1.650 kg (solids concentration: 30 wt%) of a silicone resin (YSR3022 available from Momentive Performance Materials Japan LLC.), 2.805 kg of toluene, 0.495 kg of a high-silica zeolite (HiSiv 3000 available from UNION SHOWA K.K.), 0.0495 kg of a silicone curing catalyst (YC6831 available from Momentive Performance Materials Japan LLC.), and 0.0495 kg of acetylacetone as a curing retardant. Next, the coating liquid was applied onto a porous support (RS-50 available from Nitto Denko Corporation) having a thickness of 150 $\mu$m to obtain a coating (thickness: 500 $\mu$m). The coating was heated at 90°C for 4 minutes and then dried to produce a separation functional layer having a thickness of 50 $\mu$m. The weight ratio between the silicone resin and the high-silica zeolite was 50:50 in the separation functional layer. Thus, a separation membrane was obtained.

(Measurement Example 1)

The above separation membrane was cut into a size of 75 mm in diameter to obtain a flat membrane specimen. This specimen was placed in a batch-type membrane separation device (a cell). A liquid mixture (a feed liquid) composed of n-butanol (BuOH) and water was supplied to the feed space of this cell. The content of the BuOH in the liquid mixture was 0.083 wt%.

Next, the cell was immersed in a water bath to adjust the temperature of the liquid mixture to 30°C. Next, while the liquid mixture was being stirred using a stirrer provided in the cell, the permeation space was decompressed to 1.5 kPa. Thereby, the liquid mixture permeated through the separation membrane and a permeated fluid that was a gas was obtained. The permeated fluid that was a gas was cooled by a freezing trap using liquid nitrogen so that the permeated fluid was condensed.

Next, the permeated fluid that was a liquid was taken out during a period between 0 to 60 minutes after the decompression in the permeation space was started. The permeated fluid that was a liquid was taken out every 60 minutes after the permeated fluid was firstly taken out so that three samples of the permeated fluid were obtained in total. The composition of each of the samples was analyzed by gas chromatography to measure the content of the BuOH in each of the samples. The average of the measured values obtained was regarded as the content of the BuOH in the permeated fluid. In Measurement Example 1, the content of the BuOH in the permeated fluid was 2.6 wt%.

(Measurement Examples 2 to 5)

Measurement Examples 2 to 5 were conducted in the same manner as in Measurement Example 1, except that the contents of the BuOH in the liquid mixtures were changed to 0.407 wt%, 0.740 wt%, 1.188 wt%, and 1.553 wt%, respectively. In Measurement Examples 2 to 5, the contents of the BuOH in the permeated fluids were 11.9 wt%, 22.0 wt%, 26.6 wt%, and 32.1 wt%, respectively.

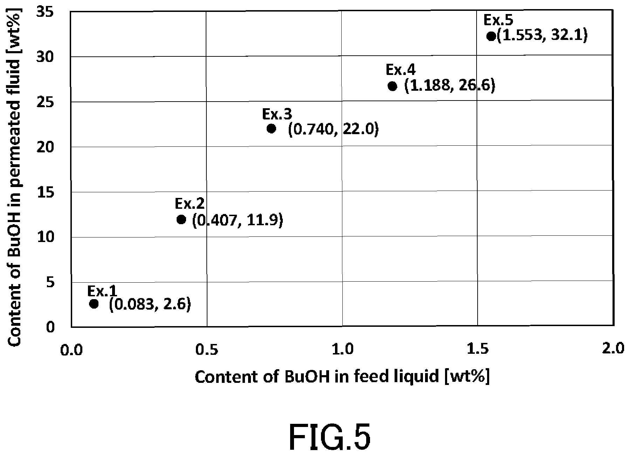

FIG. 5 shows the results of Measurement Examples 1 to 5. FIG. 5 reveals that the content of the BuOH in the liquid mixture correlates with the content of the BuOH in the permeated fluid. From these results, it is inferred that it is possible to inhibit the content of the BuOH in the permeated fluid from decreasing to about 10 wt% or less by, for example, performing the above-mentioned pressurizing procedure when the content of the BuOH in the aqueous solution to be supplied to the membrane separation device is below the first set value that is set within a range of 0.3 wt% to 0.5 wt% during operation of the membrane separation device used in each of the Measurement Examples 1 to 5.

(Measurement Example 6)

A spiral membrane element (a membrane separation device) as shown in FIG. 4 was produced using the above-mentioned separation membrane. The membrane element had a length of 300 mm and an outer diameter of 63 mm. The permeation-side flow passage material in the membrane element had a thickness of 250 $\mu$m. The supply-side flow passage material had a thickness of 850 $\mu$m. The separation membrane in the membrane element had a membrane area of 0.4 m$^2$.

Next, the produced membrane element was operated by the following method. First, a liquid mixture (a feed liquid) composed of isopropyl alcohol (IPA) and water was supplied to the feed space of the membrane element at a flow rate of approximately 100 g/min. Specifically, the liquid mixture was circulated at the above flow rate between the tank storing the liquid mixture and the membrane element. Note that the content of the IPA in the liquid mixture hardly changed during operation of the membrane element, and it was 0.26 wt%.

Next, the temperature of the liquid mixture was adjusted to 30°C by adjusting the temperatures of the tank storing the liquid mixture, pipes, a vessel accommodating the membrane element, etc. Next, the space (the permeation space) inside the central tube of the membrane element was decompressed to 1.5 kPa. Thereby, the liquid mixture permeated through the separation membrane, and a permeated fluid that was a gas was obtained. The permeated fluid that was a gas was cooled using a Dimroth condenser (the condensation unit) through which a -20°C refrigerant was circulated so that the permeated fluid was condensed. Note that considering the influence of the room temperature (25°C), the Dimroth condenser and a pipe for refrigerant circulation connected to the condenser were wrapped with a heat insulating material to avoid being in contact with outside air as much as possible.

The decompressing procedure was ended in 30 minutes after the decompression in the permeation space was started, and the inside of the Dimroth condenser was observed visually. As a result, the permeated fluid was frozen inside the condenser. The frozen permeated fluid was thawed to obtain the permeated fluid that was a liquid. The composition of the permeated fluid was analyzed by gas chromatography to measure the content of the IPA in the permeated fluid. In Measurement Example 6, the content of the IPA in the permeated fluid was 1.74 wt%.

(Measurement Examples 7 to 8)

Measurement Examples 7 and 8 were conducted in the same manner as in Measurement Example 6, except that the contents of the IPA in the liquid mixtures were changed to 1.2 wt% and 3.03 wt%, respectively. In Measurement Examples 7 and 8, the freezing of the permeated fluid was not observed inside the Dimroth condenser. In Measurement Examples 7 and 8, the contents of the IPA in the permeated fluids were 14.55 wt% and 22.26 wt%, respectively.

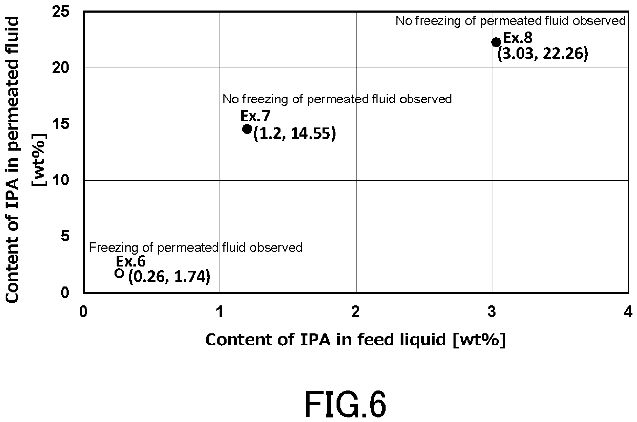

FIG. 6 shows the results of Measurement Examples 6 to 8. FIG. 6 reveals that the content of the IPA in the liquid mixture correlates with the content of the IPA in the permeated fluid. From these results, it is inferred that it is possible to inhibit the content of the IPA in the permeated fluid from decreasing to about 14 wt% or less as well as to inhibit the permeated fluid from being frozen in the condenser by, for example, performing the abovementioned pressurizing procedure when the content of the IPA in the aqueous solution to be supplied to the membrane separation device is below the first set value that is set within a range of 1.2 wt% to 3.0 wt% during operation of the membrane separation device used in each of the Measurement Examples 6 to 8.

INDUSTRIAL APPLICABILITY

The membrane separation system of the present embodiment is suitable for separating an aqueous solution, such as a fermented liquid, in which the content of an organic compound fluctuates.

**Claims**

1. A membrane separation system comprising a membrane separation device, wherein

   the membrane separation device has a separation membrane, and a feed space and a permeation space separated from each other by the separation membrane,
   when an aqueous solution containing a volatile organic compound is supplied to the feed space and the permeation space is decompressed, the separation membrane separates the aqueous solution into a permeated fluid having a content of the organic compound higher than that in the aqueous solution and a non-permeated fluid having a content of the organic compound lower than that in the aqueous solution, and
   the membrane separation system performs a pressurizing procedure that increases a pressure in the permeation space when at least one condition selected from the group consisting of conditions (A1) to (A3) below is satisfied:

      (A1) A content of the organic compound in the aqueous solution to be supplied to the feed space is below a first set value;
      (A2) A content of the organic compound in the permeated fluid is below a second set value;
      (A3) A content of the organic compound in the non-permeated fluid is below a third set value.

2. The membrane separation system according to claim 1, wherein after performing the pressurizing procedure, the membrane separation system performs a decompressing procedure that decreases the pressure in the permeation space when at least one condition selected from the group consisting of conditions (B1) to (B3) below is satisfied:

      (B1) The content of the organic compound in the aqueous solution to be supplied to the feed space is above a fourth set value;
      (B2) The content of the organic compound in the permeated fluid is above a fifth set value;
      (B3) The content of the organic compound in the non-permeated fluid is above a sixth set value.

3. The membrane separation system according to claim 1 or 2, further comprising a tank for storing the aqueous solution to be supplied to the membrane separation device.

4. The membrane separation system according to claim 3, further comprising a non-permeated fluid discharge passage connected to the membrane separation device and configured to discharge the non-permeated fluid from the membrane separation device, wherein
the non-permeated fluid discharge passage is connected to the tank.

5. The membrane separation system according to claim 3, wherein

   the organic compound is a fermented product generated by a microorganism, and
   the tank is a fermenter for generating the organic compound.

6. The membrane separation system according to claim 1, further comprising a separation tank for phase-separating the permeated fluid that is a liquid into an aqueous phase containing water as a main component and an organic phase having a content of the organic compound higher than that in the aqueous phase.

7. The membrane separation system according to claim 1, wherein the separation membrane has a separation functional layer including a hydrophobic material.

8. The membrane separation system according to claim 7, wherein the hydrophobic material includes a compound having a siloxane bond.

9. The membrane separation system according to claim 7, wherein the separation functional layer includes a matrix including the hydrophobic material and a filler dispersed in the matrix.

10. The membrane separation system according to claim 9, wherein the filler includes zeolite.

11. The membrane separation system according to claim 1, wherein the organic compound is at least one selected from the group consisting of an alcohol, ketone, and ester.

12. The membrane separation system according to claim 1, wherein the organic compound is n-butanol.

13. A method for operating a membrane separation device having a separation membrane, and a feed space and a permeation space separated from each other by the separation membrane, wherein

   when an aqueous solution containing a volatile organic compound is supplied to the feed space and the permeation space is decompressed, the separation membrane separates the aqueous solution into a permeated fluid having a content of the organic compound higher than that in the aqueous solution and a non-permeated fluid having a content of the organic compound lower than that in the aqueous solution, and
   the operating method comprises performing a pressurizing procedure that increases a pressure in the permeation space when at least one condition selected from the group consisting of conditions (A1) to (A3) below is satisfied:

   (A1) A content of the organic compound in the aqueous solution to be supplied to the feed space is below a first set value;
   (A2) A content of the organic compound in the permeated fluid is below a second set value;
   (A3) A content of the organic compound in the non-permeated fluid is below a third set value.

FIG.1

FIG.2

FIG.3

FIG.4

FIG.5

FIG.6

**INTERNATIONAL SEARCH REPORT**

| International application No. |
| --- |
| **PCT/JP2023/008428** |

**A. CLASSIFICATION OF SUBJECT MATTER**

***B01D 61/36***(2006.01)i; ***B01D 71/70***(2006.01)i
FI: B01D61/36; B01D71/70

According to International Patent Classification (IPC) or to both national classification and IPC

**B. FIELDS SEARCHED**

Minimum documentation searched (classification system followed by classification symbols)

B01D61/00-71/82; C07B63/00; C07C7/144; C12H3/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)


**C. DOCUMENTS CONSIDERED TO BE RELEVANT**

| Category* | Citation of document, with indication, where appropriate, of the relevant passages | Relevant to claim No. |
| --- | --- | --- |
| A | JP 2021-181042 A (ESEP INC) 25 November 2021 (2021-11-25) | 1-13 |
| A | JP 2020-11227 A (BENIT M CO LTD) 23 January 2020 (2020-01-23) | 1-13 |
| A | JP 2009-262108 A (STAC CO LTD) 12 November 2009 (2009-11-12) | 1-13 |
| A | JP 9-103654 A (NOK CORP) 22 April 1997 (1997-04-22) | 1-13 |
| A | JP 2015-127026 A (NGK INSULATORS LTD) 09 July 2015 (2015-07-09) | 1-13 |
| A | JP 5-169051 A (MITSUBISHI RAYON CO LTD) 09 July 1993 (1993-07-09) | 1-13 |
| A | JP 2016-73940 A (KURITA WATER IND LTD) 12 May 2016 (2016-05-12) | 1-13 |
| A | US 2003/0183338 A1 (KASHKOUSH et al.) 02 October 2003 (2003-10-02) | 1-13 |
| A | WO 2020/084996 A1 (NITTO DENKO CORP) 30 April 2020 (2020-04-30) | 1-13 |

☐ Further documents are listed in the continuation of Box C.   ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **09 May 2023** | **23 May 2023** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

| Patent document cited in search report | | | Publication date (day/month/year) | Patent family member(s) | | | Publication date (day/month/year) |
|---|---|---|---|---|---|---|---|
| JP | 2021-181042 | A | 25 November 2021 | (Family: none) | | | |
| JP | 2020-11227 | A | 23 January 2020 | US WO EP | 2021/0046424 2020/017729 3804840 | A1 A1 A1 | |
| JP | 2009-262108 | A | 12 November 2009 | (Family: none) | | | |
| JP | 9-103654 | A | 22 April 1997 | (Family: none) | | | |
| JP | 2015-127026 | A | 09 July 2015 | (Family: none) | | | |
| JP | 5-169051 | A | 09 July 1993 | (Family: none) | | | |
| JP | 2016-73940 | A | 12 May 2016 | (Family: none) | | | |
| US | 2003/0183338 | A1 | 02 October 2003 | JP WO EP | 2004-528971 2002/083278 1385607 | A A1 A1 | |
| WO | 2020/084996 | A1 | 30 April 2020 | US EP TW | 2021/0394129 3871759 202031725 | A1 A1 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010161987 A